Europäisches Patentamt

European Patent Office

Office européen des brevets

(11) Veröffentlichungsnummer: **0 123 175**
**B1**

(12) **EUROPÄISCHE PATENTSCHRIFT**

(45) Veröffentlichungstag der Patentschrift:
17.09.86

(21) Anmeldenummer: **84103693.2**

(22) Anmeldetag: **04.04.84**

(51) Int. Cl.⁴: **A 61 B 17/22**

(54) **Katheter zum Entfernen von Steinen aus dem Nieren- und Harnleiterbereich.**

(30) Priorität: **16.04.83 DE 3313895**
**09.03.84 DE 3408661**

(43) Veröffentlichungstag der Anmeldung:
**31.10.84 Patentblatt 84/44**

(45) Bekanntmachung des Hinweises auf die Patenterteilung:
**17.09.86 Patentblatt 86/38**

(84) Benannte Vertragsstaaten:
**AT CH DE FR GB IT LI NL**

(56) Entgegenhaltungen:
**DE - A - 2 436 352**
**DE - A - 2 829 159**
**DE - B - 1 170 113**
**FR - A - 1 460 776**
**US - A - 3 903 892**

(73) Patentinhaber: **Korth, Knut, Dr., Im Hau 16,**
**D-7802 Merzhausen (DE)**
Patentinhaber: **Anglomed GmbH Instrumente für**
**medizinische Diagnostik, Pforzheimer Strasse 94,**
**D-7505 Ettlingen (DE)**

(72) Erfinder: **Korth, Knut, Dr., Im Hau 16, D-7802 Merzhausen**
**(DE)**

(74) Vertreter: **Schmitt, Hans, Dipl.-Ing. et al,**
**Dreikönigstrasse 13, D-7800 Freiburg (DE)**

ACTORUM AG

**Beschreibung**

Die Erfindung betrifft einen Katheter zum Entfernen von Steinen aus dem Nieren- bzw. Harnleiterbereich, der aus einem Führungsrohr gebildet ist, in dem zur Bildung einer Schlinge zum Hintergreifen eines Steines mindestens ein Draht längsverschiebbar geführt ist, wobei das Führungsrohr beim inneren Ende mindestens eine Austrittsstelle für einen Draht aufweist.

Es ist bereits die sogenannte Dormia-Schlinge bekannt, bei der mehrere Drähte in dem Katheter geführt sind. Am inneren Ende weisen diese eine Eigenspannung zur Bildung einer Korbform auf und sind, am Korbende eine Spitze bildend, miteinander verbunden. Diese Verbindungsstelle bedingt eine erhebliche Verletzungsgefahr beim Manipulieren mit dieser Schlinge. Insbesondere bei im Bereich von einem Nierenkelch sitzenden Nierensteinen und den dann entsprechend beengten Platzverhältnissen ist bei dieser Schlinge eine besonders grosse Verletzungsgefahr vorhanden. Da die Verbindungsspitze der Dormia-Schlinge das äusserste Ende bildet, lässt sich der eigentliche Schlingenkorb nur bis zu einem der Länge der Verbindungsstelle entsprechenden Abstand in eine Nierenhöhlung, insbesondere in einen Nierenkelch einführen. Auch der für diese Schlinge insgesamt erforderliche Platzbedarf ist häufig bei in einem Nierenkelch sitzendem Stein nicht vorhanden, so dass in solchen Fällen diese Schlinge praktisch nicht eingesetzt werden kann. Insgesamt eignet sich die Dormia-Schlinge somit nur schlecht zum Hintergreifen und Manipulieren insbesondere bei einem Nierenkelchstein, so dass das Instrument unbefriedigend ist.

Es ist aus der DE-B-1 170 113 die sogenannte Zeiss-Schlinge bekannt, bei der ein im Inneren geführter Draht als Hilfsmittel zur Bildung einer Schlinge am inneren Ende des Katheters verwendet wird. Der Draht ist dabei mit Abstand zur inneren Spitze des Katheters seitlich bewegbar herausgeführt und fest mit dem äusseren Ende des Katheters verbunden. Durch Zug am Draht wird das Katheterende gebogen und zu einer Schlinge geformt. Nachteilig ist hierbei, dass der minimale, leichte Schlingenquerschnitt durch den Abstand der Austrittsstelle vom Ende des Katheters bestimmt wird. Ausserdem muss zum Entfernen eines Harnleiter-Steines der Katheter in gestreckter Form an dem Stein vorbei mit seinem inneren Ende bis etwa in den Bereich des Nierenbeckens vorgeschoben werden und kann erst dort zu einer Schlinge umgebogen werden. Anschliessend wird der Katheter zurückgebogen, wobei versucht wird, den Harnleiter-Stein zu erfassen. Nachteilig ist dabei, dass einerseits die Schlingenbildung nur in einer entsprechend grossen Höhlung möglich ist und praktisch nicht im Bereich des Steines. Somit ergibt sich im Bereich des Steines auch praktisch keine Manipulierbarkeit mit dieser Zeiss-Schlinge. Ausserdem ist in nachteiliger Weise praktisch keine Drainage der Niere möglich, so dass Komplikationen durch mangelhaften Urinfluss aus der Niere, wie kolikartige Schmerzen und Fieber auftreten können.

Aufgabe der vorliegenden Erfindung ist es, einen Katheter der eingangs erwähnten Art zu schaffen, der auch im Bereich des zu entfernenden Steines gut handhabbar und die Schlinge als aktive Schlinge dort auch ausfahrbar und manipulierbar ist und wobei das Einführen des Katheters auch unter beengten Verhältnissen, insbesondere bei der Harnleitermündung in die Blase bzw. beim Vorbeiführen an dem zu entfernenden Stein gut möglich ist. Auch ein in einem Nierenkelch sitzender Nierenstein soll gut erfasst und entfernt werden können. Es besteht darüber hinaus die allgemeinere Aufgabe, das Erfassen eines Steines im Nieren- und Harnleiterbereich unter weitestgehender Vermeidung einer Verletzungsgefahr zu verbessern.

Zur Lösung dieser Aufgabe wird vorgeschlagen, dass ein Ende eines jeden Drahtes gegenüber seiner Austrittsstelle mit Abstand versetzt am Führungsrohr befestigt ist, so dass beim Längsverschieben eines Drahtes eine aus dem Draht bestehende Schlinge gebildet wird. Die Schlingenbildung kann dadurch direkt bei dem zu erfassenden Stein erfolgen, da das Führungsrohr bzw. der Katheter seine gestreckte Form etwa beibehalten kann und lediglich die Schlinge ganz nach Bedarf ausgefahren wird. Da das am weitesten vordringende Ende der Vorrichtung durch die gebildete Schlinge gebildet ist, besteht auch in beengten Bereichen praktisch keine Verletzungsgefahr. Die Vorrichtung lässt sich somit besonders gut auch zum Entfernen von Nierensteinen aus dem Bereich der Nierenkelche einsetzen.

Zweckmässigerweise bildet die innere Kathetermündung die Austrittsstelle für den Schlingen-Draht. Bei dieser Austrittsstelle ist der Draht zur Bildung einer Schlinge hin- und herverschiebbar. Diese Ausführungsform ist besonders einfach realisierbar.

Vorteilhafterweise ist die Befestigungsstelle des Schlingendrahtes am Führungsrohr aussenseitig vorgesehen und weist von der Kathetermündung bzw. von der Austrittsstelle einen Abstand von 5 mm bis 40 mm, vorzugsweise 20 mm auf. Durch diese Ausbildung lässt sich eine knickfreie, den gegebenen räumlichen Verhältnissen weitgehend angepasste Schlinge bilden. Die Befestigungsstelle kann in Abhängigkeit insbesondere von der Elastizität des Katheterendes und auch des Drahtes gewählt werden. Zur Erzielung einer in sich etwas steiferen Schlinge wird dabei der Abstand etwas kleiner gewählt, während für eine in sich etwas weichere Schlinge der grössere Abstand vorgesehen ist.

Gegebenenfalls können mehrere, vorzugsweise voneinander unabhängig verschiebbare Schlingen-Drähte vorgesehen sein, wobei diese Schlingen-Drähte beim Befestigungsende am Führungsrohr gegebenenfalls miteinander verbunden sind und sich die Verbindungsstelle im wesentlichen innerhalb des Katheters befindet. Dadurch wird die Möglichkeit des Ergreifens von Steinen insbesondere bei schwierigen Verhältnissen, verbessert. Ausserdem wird die Gefahr, dass ein Stein nach dem Erfassen wieder entgleitet, vermindert.

Nach einer Weiterbildung ist vorgesehen, dass der Schlingen-Katheter mit einem Drainage-Katheter kombiniert ist und die Drahtschlinge gegenüber dem inneren Drainage-Katheter-Ende zurückversetzt angeordnet ist. Diese Ausführungsform ist insbesondere zum Entfernen von Harnleiter-Steinen einsetzbar.

In vorteilhafter Weise wird dabei vermieden, dass ausser einem Schlingen-Katheter auch noch ein zusätzlicher Katheter am Stein zur Drainage der Niere vorbeigeführt werden muss. Auch hier ist im Schlingenbereich eine aktive Manipulierbarkeit zum Erfassen des Steines gegeben.

Eine abgewandelte Ausführungsform sieht vor, dass die für den Schlingendraht vorgesehene Austrittsöffnung sich seitlich am Führungsrohr befindet und einen grösseren Abstand zum inneren Ende des Katheters bzw. Führungsrohres aufweist als die Befestigungsstelle des Schlingendrahtes am Führungsrohr. Dadurch wird vermieden, dass sich eine Umlenkstelle des Drahtes in Form einer Aufbauchung oder dgl. bildet. Bei eingezogenem Schlingendraht kann sich somit der ausserhalb des Katheters befindliche Draht-Abschnitt im wesentlichen glatt an die Aussenseite des Katheters anlegen und bildet somit keine störende, vorstehende Aufbauchung. Dadurch ist insbesondere auch das Passieren des Katheters mit dem Schlingenbereich am Harnleiterstein vorbei wesentlich einfacher auch unter beengten Verhältnissen möglich.

Zweckmässigerweise ist dabei das Befestigungsende des Schlingendrahtes gelenkartig mit dem Führungsrohr verbunden. Durch dieses Verbindungsgelenk ist eine im Verbindungsbereich weitgehend biegefreie Einstellung des Drahtes auf die jeweilige Schlingengrösse gegeben, wobei insbesondere auch bei ganz eingezogenem Draht, wo er an dem Katheter-Führungsrohr aussen aliegt, keine Biegung bzw. elastische Verformung im Befestigungsbereich vorhanden ist. Vielmehr kann sich der Draht flach und dicht an den Aussenmantel des Katheters anlegen und stört somit insbesondere beim Einführen des Katheters nicht.

Eine Ausführungsform sieht vor, dass das Befestigungsende des Schlingendrahtes eine Umbiegung zum Einhängen in eine Befestigungsöffnung aufweist. Diese Einhängeverbindung bildet ein lösbares Verbindungsgelenk, wodurch einerseits die Montage des Schlingendrahtes vereinfacht und dieser auch gegebenenfalls auf einfache Weise entnommen werden kann. Zusätzliche Ausgestaltungen der Erfindung sind in den weiteren Unteransprüchen aufgeführt. Nachstehend sind Ausführungsbeispiele der Erfindung mit ihren wesentlichen Einzelheiten anhand der Zeichnung noch näher erläutert.

Es zeigt in unterschiedlichen Massstäben:

Fig. 1 eine Teilseitenansicht eines erfindungsgemässen Katheters mit in unterschiedlichen Stellungen befindlicher Schlinge,

Fig. 2 eine schematische Schnittdarstellung einer Niere mit in einem Nierenkelch befindlichem Stein sowie einer darumgelegten Schlinge eines teildargestellten Schlingenkatheters,

Fig. 3 eine perspektivische Ansicht des inneren Endes eines Katheters mit zwei zueinander versetzt angeordneten Schlingen,

Fig. 4 eine Ansicht des inneren Endes eines Katheters mit zwei Drähten und etwa in Parallellage befindlichen Schlingen,

Fig. 5 eine etwa Fig. 3 und 4 entsprechende Ansicht, hier jedoch mit einem flach ausgebildeten Schlingen-Draht,

Fig. 6 eine Stirnseitenansicht eines Katheters mit bandförmig flach ausgebildetem, etwa in Ruhelage befindlichem Schlingen-Draht,

Fig. 7 eine etwa Fig. 6 entsprechende Stirnseitenansicht, hier jedoch mit einem im Querschnitt runden Schlingen-Draht,

Fig. 8 eine Stirnseitenansicht ähnlich Fig. 6 und 7, hier jedoch mit kreuzartig zueinander angeordneten, einerseits flachen, andererseits runden Schlingen-Drähten,

Fig. 9 eine abgewandelte Ausführungsform eines erfindungsgemässen Katheters in Verbindung mit einem Drainage-Katheter,

Fig. 10 eine Teilseitenansicht eines abgewandelten Katheters mit einem Drainage-Katheter mit in unterschiedlichen Stellungen befindlichem Schlingen-Draht,

Fig. 11 einen Teillängsschnitt eines Katheters nach Fig. 10 mit einem eine Schlinge bildenden Draht,

Fig. 12 eine etwa Fig. 11 entsprechende Darstellung, hier jedoch mit Lage des Schlingendrahtes in eingezogenem Zustand,

Fig. 13 eine abgewandelte Ausführungsform des Befestigungsendes eines Schlingendrahtes und

Fig. 14 eine wiederum abgewandelte Ausführungsform des Befestigungsendes eines Schlingendrahtes.

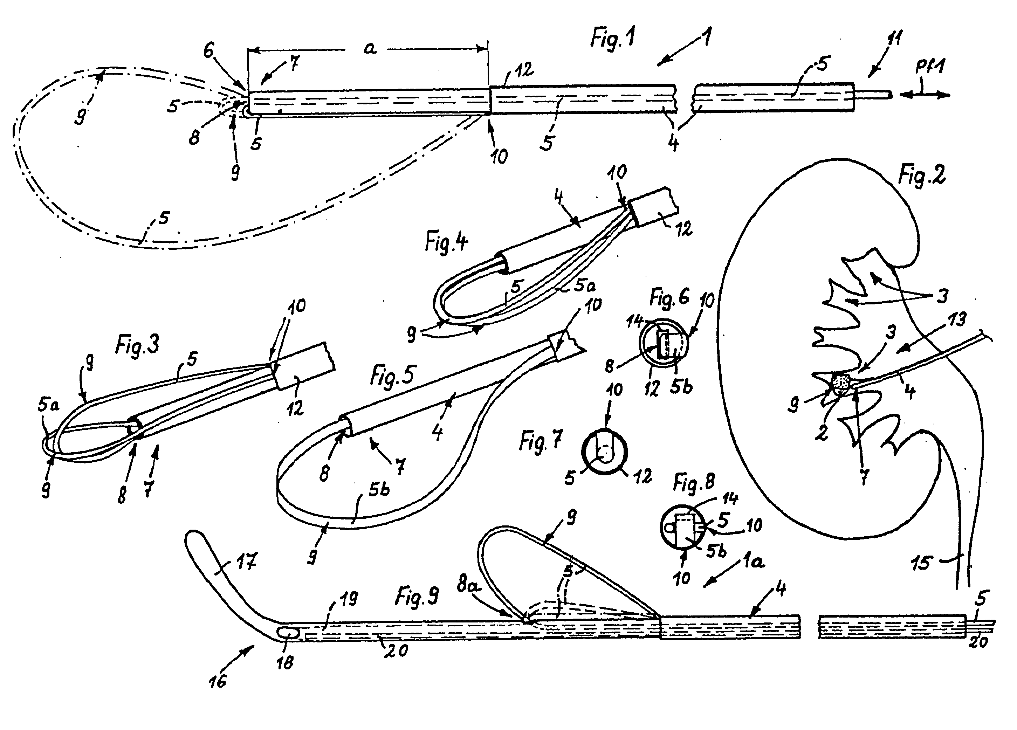

Ein in den Fig. 1 bis 8 gezeigter Schlingen-Katheter 1 dient insbesondere zum Entfernen von Nierensteinen 2 im Nierenbereich und dabei insbesondere im Bereich von Nierenkelchen 3 (vgl. Fig. 2).

Bei einer Nierensteinoperation wird der Schlingenkatheter 1 unter Zuhilfenahme eines Pyeloskops durch dessen in seinem Schaft befindlichen Arbeitskanal in den Nierenbereich eingebracht. Der Schlingenkatheter 1 weist im wesentlichen ein Führungsrohr 4 auf, in dem ein Draht 5 längsverschiebbar gemäss dem Doppelpfeil Pf1 (Fig. 1) geführt ist. Die Kathetermündung 6 beim inneren Ende 7 bildet hier die Austrittsstelle 8 für den Draht 5, der sich dann je nach Position von diesem als kleinere oder grössere Schlinge 9 zu einer Befestigungsstelle 10 fortsetzt. Diese Befestigungsstelle ist im Ausführungsbeispiel aussenseitig am Führungsrohr 4 vorgesehen. Sie befindet sich mit Abstand zu der Austrittsstelle 8 zurückversetzt an dem Katheter. Der Abstand a dieser Befestigungsstelle 10 von der Austrittsstelle 8 kann je nach den Erfordernissen zwischen 5 mm und 40 mm liegen und ist vorzugsweise bei etwa 20 mm Abstand vorgesehen.

In Fig. 1 ist die Schlinge 9 in drei verschiedenen Stellungen eingezeichnet. Durchgehend liniert ist dabei die Einführstellung gezeichnet, wo der Draht 5, ausgehend von der Befestigungsstelle 10 bis zur Austrittsstelle 8 eng an der Katheter-Aussenseite anliegt. Durch geringfügiges Einschieben des Drahtes 5 von dem äusseren Bedienende 11 her bildet sich die kleine, strichlinierte Schlinge 9, während durch weiteres Einschieben des Drahtes 5 eine praktisch beliebig grosse Schlinge 9, wie dies strichpunktiert angedeutet ist, gebildet werden kann. Bei der etwas grösseren Schlingenbildung ist auch erkennbar, dass der Endbereich des Drahtes 5, ausgehend von der Befestigungsstelle 10, sich durch die Schlingenbildung vom Führungsrohr 4 abgehoben hat.

Die Befestigung des Endes des Drahtes 5 am Führungsrohr 4 kann durch eine Katheter-Ummantelung 12 erfolgen.

Durch die erfindungsgemässe Ausbildung des Schlingen-Katheters 1 wird nicht mehr wie bisher der Endbereich des Führungsrohres 4 unter Zuhilfenahme des Drahtes 5 als Zugelement zu einer Schlinge umgebogen, sondern der Draht 5 selbst wird hier zum aktiven Teil und bildet selbst die Schlinge 9, während das Ende des Führungsrohres 4 im wesentlichen unverformt oder nur wenig verformt wird. Dadurch ergeben sich beim Manipulieren zum Erfassen eines Nierensteines oder dgl. wesentlich günstigere Platzverhältnisse, so dass dicht sitzende Steine noch erfasst werden können.

Insbesondere ist dadurch auch die Möglichkeit gegeben, im Bereich von Nierenkelchen 3 (Fig. 2) sitzende Steine zu erfassen, ohne dass dabei die Verletzungsgefahr wie bei der sogenannten Dormia-Schlinge besteht. Zum Erfassen eines in einem Nierenkelch 3 befindlichen Nierensteines 2 wird der Schlingenkatheter 1 mit seinem inneren Ende 7 bei in der Regel noch eingezogener Schlinge 9, bis an den Nierenstein 2 herangeführt. Durch Ausfahren der Schlinge 9, die dabei mit ihrem vergleichsweise dünneren Draht 5 an dem Nierenstein 2 vorbeigelangt, z.B. durch anschliessendes Verdrehen oder dgl. Manipulieren der Schlinge 9, wird diese dann um den Nierenstein 2 herumgelegt. Von wesentlicher Bedeutung ist dabei, dass durch die glatte, keine vorstehenden Teile aufweisende Schlinge 9, auch bei beengten Verhältnissen, wie sie insbesondere in einem Nierenkelch 3 herrschen, die Verletzungsgefahr des umgrenzenden Nierengewebes und dgl. weitgehend vermindert wird.

Nach dem Ergreifen des Nierensteines 2 kann dieser aus dem Nierenkelch 3 herausgezogen und z.B. ins Nierenbecken 13 zur weiteren Aufarbeitung transportiert werden.

Erwähnt sei noch, dass, wie vorbeschrieben, bei beengten Verhältnissen nur die Schlinge 9 an dem zu erfassenden Nierenstein 2 vorbeigeschoben wird. Falls entsprechender Platz vorhanden ist, kann jedoch auch der Katheter 4 selbst am Nierenstein 2 vorbeigeschoben, dann die Schlinge ausgefahren und der Stein erfasst werden. Man hat somit je nach den gerade vorhandenen Verhältnissen verschiedene Möglichkeiten, mit dem erfindungsgemässen Schlingen-Katheter einen Nierenstein 2 zu erfassen. Der besondere Vorteil liegt darin, dass die Schlingenbildung auch bei beengten Platzverhältnissen ohne Verletzungsgefahr erfolgen kann. Sowohl das Führungsrohrende als auch die Schlinge 9 können dabei praktisch unabhängig voneinander positioniert bzw. manipuliert werden.

Die Fig. 3 und 4 zeigen noch Ausführungsbeispiele, bei denen mehrere Schlingendrähte 5, 5a vorgesehen sind. Durch eine versetzte Anordnung der Befestigungsstellen 10 der Enden der Drähte 5, 5a und auch durch entsprechendes Verdrehen von diesen beim Manipulieren, können gegeneinander in ihrer Schlingenebene versetzte Schlingen 9 und damit eine etwa körbchenförmige Ausbildung durch diese beiden Schlingen erzielt werden.

Bei der Ausführungsform gemäss Fig. 4 sind zwei Schlingen-Drähte 5, 5a etwa parallel zueinander geführt und an einer gemeinsamen Befestigungsstelle 10 mit dem Führungsrohr 4 verbunden. Auch diese Ausführungsform kann in bestimmten Anwendungsfällen zum sicheren Erfassen eines Nierensteines 2 vorteilhaft sein. Denkbar ist bei dieser Ausführungsform auch, dass z.B. der Draht 5 durch Verdrehen im Schlingenbereich etwas von der durch den Draht 5a gebildeten Schlinge abgehoben werden kann. In dieser Lage könnten z.B. beide Schlingen an gegenüberliegenden Seiten eines Steines vorbeigeführt und hinter dem Stein dann wieder durch Verdrehen der Drähte etwas zueinander bewegt werden, so dass der Nierenstein dann erfasst werden kann.

Neben einer zweidrähtigen Ausführung, wie in Fig. 3 und 4 gezeigt, können auch noch mehr als zwei Drähte gegebenenfalls vorgesehen sein. Erwähnt sei noch, dass die einzelnen Drähte 5, 5a usw. vorzugsweise voneinander unabhängig verschiebbar sind, so dass eine besonders gute Anpassung an die jeweiligen Gegebenheiten und insbesondere auch das Erfassen von Nierensteinen unter schwierigen Bedingungen möglich ist.

Neben den im Querschnitt etwa kreisförmigen Schlingen-Drähten kommen auch solche mit einem von der Kreisform abweichenden Querschnitt in Frage. Der Querschnitt kann dabei in etwa rechteckig oder oval, ellipsenförmig oder dgl. ausgebildet sein. Man erreicht dadurch eine Vorzugsbiegeebene, durch die bestimmte Positioniermöglichkeiten für die Schlinge verbessert sein können. Fig. 5 zeigt eine solche Schlinge 9 mit einem etwa bandförmigen Draht 5b. Wegen der flachen Ausführung des Drahtes 5b könnte dieser auch in sehr engen Radien gebogen werden, so dass eine Befestigung des Drahtendes in der Nähe oder gegebenenfalls sogar bei der Austrittsstelle 8 denkbar ist.

Die Stirnseitenansichten gemäss Fig. 6 bis 8 zeigen unterschiedliche Ausführungsformen, wobei jedoch alle Schlingen sich etwa in Ausgangsstellung, d.h. am Führungsrohr 4 etwa anliegend befinden. In Fig. 6 ist auch angedeutet, dass bei der Austrittsstelle 8 eine etwa dem Querschnitt des Drahtes 5b entsprechende Führungsprofilierung 14 aufweist. Durch diese Führung des Drahtes 5b insbesondere im Bereich der Austrittsstelle 8, ergibt sich jeweils in einer durch die Führung und die Befestigungsstelle 10 vorgegebenen Ebene beim Herausschieben des Drahtes 5b eine entsprechende Schlingenbildung.

Fig. 7 zeigt in Stirnseitenansicht das innere Ende 7 des Schlingenkatheters 1 gemäss Fig. 1.

Bei der Stirnseitenansicht gemäss Fig. 8 sind die Austrittsstellen zweier Schlingendrähte 5, 5b etwa um 90° versetzt angeordnet und als Führungen durch eine entsprechende Führungsprofilierung 14 ausgebildet. Auch die Befestigungsstellen 10 weisen einen entsprechenden Versatz am Umfang des Katheters 4 um etwa 90° auf. In diesem Ausführungsbeispiel, bei dem sich beim Ausfahren beider Schlingen 9 etwa die in Fig. 3 gezeigte Schlingenausbildung einstellt, ist in Kombination ein Flachdraht 5b sowie ein Runddraht 5 vorgesehen.

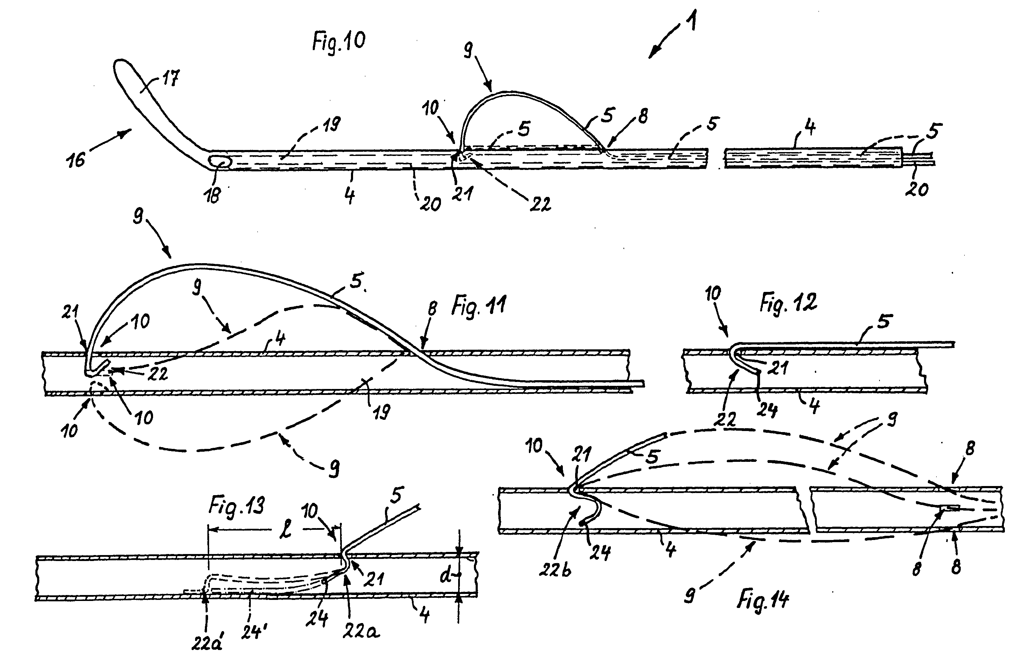

Fig. 9 zeigt eine Ausführungsvariante, bei der der erfindungsgemässe Schlingenkatheter 1 gemäss Fig. 1 mit einem Drainage-Katheter kombiniert ist.

Diese Vorrichtung 1a dient insbesondere zum Entfernen von Nieren- bzw. Harnleitersteinen aus dem Bereich des Harnleiters 15 (Fig. 2). Bei bekannten Kathetern kam es häufig vor, dass nach dem Einführen des Katheters und dem Erfassen des Harnleitersteines der Harntransport blockiert war. Häufig wurde dann versucht, durch einen Ureteren-Katheter, der neben den Schlingenkatheter gelegt wurde, eine Drainage der Niere zu erreichen. Einen zweiten Katheter neben einen Stein zu schieben ist jedoch meistens schwierig und häufig auch gar nicht möglich.

Bei der in Fig. 9 gezeigten Vorrichtung 1a ist durch die Kombination eines erfindungsgemässen Schlingen-Katheters mit einem Drainage-Katheter gleichzeitig die Möglichkeit der Drainage der Niere und auch das Erfassen eines Harnleitersteines gegeben. Die Austrittsstelle 8a für den Schlingendraht 5 und damit die gesamte Schlinge 9 ist gegenüber dem Drainage-Katheter-Ende 16 zurückversetzt. Bei diesem Drainage-Katheter-Ende 16 ist eine Abkrümmung 17 vorgesehen, durch die das Vorbeiführen des gesamten Katheters an einem Stein, insbesondere durch Drehbewegungen des Katheters, erleichtert ist. Im Übergangsbereich von dieser Abkrümmung 17 zum Drainage-Katheter ist eine Drainage-Öffnung 18 zu einer Drainage-Höhlung 19 vorgesehen. Die längs im Katheter-Führungsrohr 4 verlaufende Innenhöhlung kann gleichzeitig die Führungshöhlung für den Schlingendraht 5 und auch die Drainage-Höhlung 19 bilden. Andererseits besteht aber auch die Möglichkeit, die Drainage-Höhlung und die Führungshöhlung zumindest bereichsweise, insbesondere im Bereich der Austrittsstelle 8a voneinander getrennt zu führen. Insbesondere bleibt dadurch die Drainage-Höhlung 19 frei durchgängig und wird nicht durch den Schlingendraht 5 beeinträchtigt.

In Fig. 9 erkennt man noch einen eingeschobenen Versteifungsdraht 20, durch den das Einführen des Katheters vereinfacht wird. Nach dem Einführen des Katheters kann dieser Versteifungsdraht 20 herausgezogen werden.

Für das Führungsrohr 4 kann sowohl ein Metallspiralschlauch oder ein Kunststoffschlauch verwendet werden. Der Schlingendraht 5, 5a, 5b kann je nach den Erfordernissen aus z.B. Federstahldraht oder aber auch aus Kunststoff, z.B. einem Polyamid, bestehen.

Um eine Aufdickung des inneren Endbereiches bei anliegendem Schlingendraht zu vermeiden, kann ausgehend von der Befestigungsstelle 10 bis zur Austrittsstelle 8, 8a eine rinnenartige Tasche im Führungsrohr 8 zur mindestens teilweisen Aufnahme des Drahtes vorgesehen sein. Bei der Befestigungsstelle 10 könnte gegebenenfalls, z.B. durch eine Materialschwächung auch ein Gelenk zur leichteren Auslenkbarkeit der Schlinge 9 vorgesehen sein.

Beim äusseren Bedienende 11 kann der bzw. die Schlingen-Drähte 5, 5a, 5b gegebenenfalls noch durch eine Klemmvorrichtung oder dgl., vorzugsweise einzeln festlegbar sein. Sie können dort auch entsprechende Griffausbildungen zum besseren Manipulieren aufweisen.

Wie in Fig. 9 besteht auch die abgewandelte Ausführungsform einer Vorrichtung 1 gemäss Fig. 10 aus einer Kombination von einem Drainage-Katheter und einem Schlingen-Katheter. In Fig. 10 ist gut erkennbar, dass die Schlinge 9 gegenüber dem inneren Katheterende 16 auch hier zurückversetzt angeordnet ist. Bei diesem Katheter 1 ist nun vorgesehen, dass eine für den Schlingendraht 5 vorgesehene, seitliche Austrittsstelle 8 am Katheter einen grösseren Abstand zum Ende 16 des Katheters aufweist als die Befestigungsstelle 10 des Schlingendrahtes 5 am Führungsrohr 4 des Katheters. Durch diese Ausbildung kann sich der Schlingen-Draht 5 in eingezogenem Zustand (vgl. strichliniert Fig. 10 sowie Fig. 12) glatt an die Aussenseite des Führungsrohres 4 anlegen. Eine sonst praktisch nicht zu vermeidende Umlenkaufbauchung, die mehr oder weniger stark über den Umfang des Führungsrohres 4 vorstehen würde, wird dadurch vermieden. Insbesondere in Fig. 12 ist erkennbar, dass durch die erfindungsgemässe Anordnung und Ausbildung von Austrittsstelle und Befestigungsstelle des Drahtes eine solche Umlenkung mit entsprechender Aufbauchung vermieden wird.

Beim Befestigungsende 10 des Drahtes 5 ist hier eine gelenkartige Verbindung mit dem Katheter vorhanden, durch den sich der Draht in diesem Bereich weitgehend verformungsfrei an die jeweilige Schlingenstellung anpassen kann.

Die Befestigung des Endes des Drahtes 5 kann, wie in den Fig. 10 bis 14 erkennbar, vorzugsweise durch eine Umbiegung des Drahtendes und durch Einhängen dieser Umbiegung in eine Befestigungsöffnung 21 erfolgen. Neben einer einfachen Montage ist dadurch auch eine Möglichkeit gegeben, den Draht durch Aushängen einfach zu entnehmen.

In den in Fig. 11 und 12 gezeigten Ausführungsbeispielen ist die Umbiegung 22 hakenförmig oder gegebenenfalls U-förmig mit zurückgezogenem Hakenende ausgebildet. Ein solches Hakenende lässt sich besonders einfach formen und ergibt bereits eine gute Verbindung des Drahtes 5 mit dem Führungsrohr 4 des Katheters 1.

Eine abgewandelte Ausführungsform einer Umbiegung 22a zeigt Fig. 13. Hier ist die Umbiegung etwa L-förmig mit nach vorne weisendem Hakenende ausgebildet. Auch hier ist das Einhängen des Drahtendes besonders einfach und es ist hier auch eine erhöhte Sicherung gegen Herausspringen des Drahtendes bei einer Schlingenbildung gegeben. Strichliniert ist in Fig. 13 noch angedeutet, dass mit Abstand zu der Umbiegung 22a noch eine weitere Umbiegung 22a' vorgesehen sein kann. Der Abstand I zwischen den Umbiegungen kann z.B. 5- bis 8mal so gross sein, wie der lichte Durchmesser des Katheter-Röhrchens 4 beträgt. Die doppelte bzw. mehrfache Anordnung von Umbiegungen hat den Vorteil, dass bei einem eventuellen Herausrutschen der Umbiegung 22a aus der Befestigungsstelle 19 die zweite Umbiegung 22a' einhakt, so dass eine erhöhte Sicherheit gegeben ist.

Das freie, nach vorne gerichtete Hakenende 24 bzw. 24' ist vorzugsweise etwas verlängert ausgebildet, wie dies strichpunktiert in Fig. 13 angedeutet ist. Diese Länge I kann etwa dem 5- bis 8fachen des lichten Innendurchmessers d des Röhrchens 4 entsprechen. Auch dadurch ergibt sich bereits eine verbesserte Halterung des Drahtendes, da sich das verlängerte Hakenende an der Innenwand des Röhrchens 4 anlegen und abstützen kann.

Besonders vorteilhaft ist dabei auch die insbesondere L-förmige Umbiegung, da diese beim Zurückziehen des Drahtes, falls dieser ausgehängt hat, weit weniger zu Verletzungen innerhalb der Harnröhre und dgl. führen kann als z.B. ein gekrümmter Haken. Auch andere, ähnliche Hakenformen, z.B. gemäss Fig. 14, weisen diesen Vorteil in vergleichbarer Form auf.

Eine weitere mögliche Ausführungsform einer Umbiegung 22b zeigt Fig. 14. Diese ist etwa Z-förmig mit nach vorne weisendem Hakenende ausgebildet. Diese Ausführungsform bildet praktisch eine Kombination der in Fig. 11 und 13 gezeigten Umbiegungen, wobei hier sowohl bei Zugbelastung des Drahtes 5 als auch bei Schubbeaufschlagung zur Bildung einer Schlinge ein Herausspringen des Schlingendraht-Endes praktisch nicht möglich ist. Trotzdem kann von Hand auf einfache Weise ein Einhängen bzw. Aushaken erfolgen. Bei der Ausführungsform nach Fig. 14 ist noch vorteilhaft, wenn das Hakenende 24 in Befestigungslage etwa an der der Befestigungsöffnung 21 gegenüberliegenden Katheter-Innenwand 23 anliegt. Dadurch ergibt sich eine definierte Lage des Durchtrittsbereiches des Drahtes durch die Befestigungsöffnung 21 sowohl bei Zug- als auch bei Druckbelastung des Drahtes.

Erwähnt sei noch, dass das Befestigungsende des Schlingendrahtes 5 auch aussen am Katheter befestigt sein kann, z.B. mittels eines gegebenenfalls eine äussere Umhüllung des Katheters bildenden Schrumpfschlauches oder dgl.

Der Drainage-Katheter 1 kann neben der beim inneren Ende vorgesehenen Drainage-Öffnung 18 auch im weiteren Verlauf noch Querbohrungen aufweisen. Gegebenenfalls könnten diese dann auch gleichzeitig als Befestigungsöffnungen 21 mitverwendet werden, wobei man den Vorteil hat, die Einhängestelle in Anpassung an eine gewünschte Schlingengrösse variieren zu können.

Wie bereits vorerwähnt, kann sich der Schlingendraht 5 in eingezogener Lage praktisch glatt an die Aussenseite des Führungsrohres 4 anlegen. Da dieser, vorzugsweise aus Stahl bestehende Draht vergleichsweise dünn ist, steht über den Katheteraussenmantel kein insbesondere beim Einführen des Katheters bzw. Vorbeiführen am Stein störender Vorsprung vor. Nach einer Weiterbildung der Erfindung könnte gegebenenfalls im Bereich zwischen der Befestigungsstelle 10 des Drahtes 5 und der für ihn vorgesehenen Austrittsöffnung 8, am Katheter eine längs verlaufende, vorzugsweise etwa dem Querschnitt des Drahtes entsprechende Längsrille oder dgl. Vertiefung vorgesehen sein. Bei eingezogenem Draht 5 würde sich dieser ausserhalb des Führungsrohrbereiches liegende Teil des Drahtes dann in die Vertiefung legen können, so dass sogar der Draht 5 sich innerhalb des Umfangsquerschnittes des Katheters befindet. Dadurch wäre dann auch die Verwendung eines Schlingendrahtes aus etwas dickerem Material, gegebenenfalls auch aus Kunststoff, möglich.

In den Fig. 10 bis 14 ist eine Ausführungsform eines Katheters 1 mit jeweils einem Schlingendraht 5 gezeigt. Es können jedoch auch mehrere, vorzugsweise voneinander unabhängig verschiebbare Schlingendrähte vorgesehen sein. Diese können entweder am Umfang des Katheters verteilt befestigt sein, oder nach einer bevorzugten Ausführungsform ist vorgesehen, dass z.B. zwei bis fünf, vorzugsweise drei Schlingen-Drähte über einen Umfangsbereich zwischen 10 bis 360°, vorzugsweise 10 bis 45° verteilt angeordnet sind. In weiterer Ausbildung der Erfindung können dabei die Befestigungsenden der Schlingen-Drähte miteinander verbunden sein, wobei sich die Verbindungsstelle vorzugsweise im wesentlichen innerhalb des Führungsrohres 4 befindet. Durch diese Verbindung bleibt der Abstand der einzelnen Schlingen zueinander — in Umfangsrichtung des Katheters — etwa gleich, so dass mit diesen Schlingen praktisch ein Korb gebildet werden kann. Bei mehreren Schlingendrähten können für diese auch mehrere Austrittsöffnungen 8 und gegebenenfalls auch mehrere Befestigungsöffnungen 21 vorgesehen sein.

Fig. 11 zeigt strichliniert noch angedeutet mehrere Schlingen 9, die hier jeweils eine Befestigungsstelle 10 und eine gemeinsame Austrittsstelle 8 haben. Fig. 14 zeigt in einer anderen Ausführungsform strichliniert mehrere Schlingen 9, die gemeinsam eine Befestigungsstelle 10, jedoch getrennte, hier beispielsweise um den Umfang des Führungsrohres 4 verteilt angeordnete Austrittsstellen 8 haben. Der Katheter kann zumindest im Verhakungsbereich entsprechend formstabil zum Halten des Schlingendraht-Endes ausgebildet sein.

**Patentansprüche**

1. Katheter zum Entfernen von Steinen aus dem Nieren- bzw. Harnleiterbereich, der aus einem Führungsrohr gebildet ist, in dem zur Bildung einer Schlinge zum Hintergreifen eines Steines mindestens ein Draht längsverschiebbar geführt ist, wobei das Führungsrohr beim inneren Ende mindestens eine Austrittsstelle für einen Draht aufweist, dadurch gekennzeichnet, dass ein Ende eines jeden Drahtes (5, 5a, 5b) gegenüber seiner Austrittsstelle (8, 8a) mit Abstand versetzt am Führungsrohr (4) befestigt ist, so dass beim Längsverschieben eines Drahtes eine aus dem Draht bestehende Schlinge (9) gebildet wird.

2. Katheter nach Anspruch 1, dadurch gekennzeichnet, dass die innere Katheter-Mündung (6) die Austrittsstelle (8) für den Schlingen-Draht (5, 5a, 5b) bildet.

3. Katheter nach Anspruch 1 oder 2, dadurch gekennzeichnet, dass die Befestigungsstelle (10) des Schlingen-Drahtes am Führungsrohr (4) aussenseitig vorgesehen ist und von der Katheter-Mündung (6) bzw. von der Austrittsstelle (8, 8a) einen Abstand von 5 mm bis 40 mm, vorzugsweise 20 mm, aufweist.

4. Katheter nach einem der Ansprüche 1 bis 3, dadurch gekennzeichnet, dass mehrere, vorzugsweise voneinander unabhängig verschiebbare Schlingen-Drähte (5, 5a) vorgesehen sind, und dass diese Schlingen-Drähte beim Befestigungsende am Führungsrohr gegebenenfalls miteinander verbunden sind, wobei sich die Verbindungsstelle im wesentlichen innerhalb des Katheters befindet.

5. Katheter zum Entfernen von Harnleitersteinen, dadurch gekennzeichnet, dass der Schlingen-Katheter (1) nach einem der Ansprüche 1 bis 4 mit einem Drainage-Katheter kombiniert ist und die Draht-Schlinge (9) gegenüber dem inneren Drainage-Katheter-Ende (16) zurückversetzt angeordnet ist.

6. Katheter nach Anspruch 5, dadurch gekennzeichnet, dass ein Drainage-Kanal (19) und ein Führungskanal für den Draht (9) durch einen gemeinsamen Kanal gebildet sind.

7. Katheter nach Anspruch 5, dadurch gekennzeichnet, dass der Drainage-Kanal (19) und der Führungskanal für den Draht (5, 20) zumindest bereichsweise, insbesondere im Bereich der Austrittsstelle (8a), voneinander getrennt sind.

8. Katheter nach einem der Ansprüche 1 bis 7, dadurch gekennzeichnet, dass der bzw. die Schlingen-Drähte (5, 5a, 5b) am äusseren Bedienende (11) vorzugsweise festlegbar sind.

9. Katheter nach einem der Ansprüche 1 bis 8, dadurch gekennzeichnet, dass eine äussere Umhüllung (12) des Führungsrohres (4) zur Befestigung des inneren Draht-Endes am Führungsrohr (4) vorgesehen ist.

10. Katheter nach einem der Ansprüche 1 bis 9, dadurch gekennzeichnet, dass die für den Schlingendraht (5) vorgesehene Austrittsöffnung (8) sich seitlich am Führungsrohr (4) befindet und einen grösseren Abstand zum inneren Ende des Katheters (1) bzw. Führungsrohres (4) aufweist als die Befestigungsstelle (10) des Schlingendrahtes (5) am Führungsrohr.

11. Katheter nach Anspruch 10, dadurch gekennzeichnet, dass das Befestigungsende (10) des Schlingendrahtes (5) gelenkartig mit dem Führungsrohr (4) verbunden ist.

12. Katheter nach Anspruch 10 oder 11, dadurch gekennzeichnet, dass das Befestigungsende des Schlingendrahtes (5) eine Umbiegung (22, 22a, 22b) zum Einhängen in eine Befestigungsöffnung (21) aufweist.

13. Katheter nach Anspruch 12, dadurch gekennzeichnet, dass die Umbiegung (22) hakenförmig oder U-förmig mit zurückgebogenem Hakenende (24) ausgebildet ist.

14. Katheter nach Anspruch 12, dadurch gekennzeichnet, dass die Umbiegung (22a) L-förmig mit nach vorne weisendem Hakenende ausgebildet ist, wobei vorzugsweise das freie, nach vorne gerichtete Hakenende eine etwa dem 5- bis 8fachen des lichten Innendurchmessers (d) des Führungsrohres (4) entsprechende Länge hat, und dass gegebenenfalls mehrere, zweckmässigerweise zwei dieser Umbiegungen (22a, 22a') mit Abstand zueinander vorgesehen sind.

15. Katheter nach Anspruch 12, dadurch gekennzeichnet, dass die Umbiegung Z-förmig mit nach vorne weisendem Hakenende (24) ausgebildet ist, und dass das Hakenende (24) in Befestigungslage vorzugsweise an der der Befestigungsöffnung gegenüberliegenden Führungsrohr-Innenwand (23) anliegt.

16. Katheter nach einem der Ansprüche 1 bis 15, dadurch gekennzeichnet, dass das Befestigungsende des Schlingendrahtes (5) aussen am Führungsrohr (4) befestigt ist, z.B. mittels eines gegebenenfalls eine äussere Umhüllung des Führungsrohres bildenden Schrumpfschlauches.

17. Katheter nach einem der Ansprüche 1 bis 16, dadurch gekennzeichnet, dass der Schlingendraht (5) aus Stahldraht, gegebenenfalls aus Kunststoff besteht.

18. Katheter nach einem der Ansprüche 1 bis 17, dadurch gekennzeichnet, dass der Abstand zwischen Austrittsöffnung (8) und Befestigungsstelle (10) für den Schlingendraht (5) bzw. die Schlingen-Drähte, etwa 5 bis 40 mm, vorzugsweise 20 mm, beträgt und dass gegebenenfalls mehrere Befestigungsstellen mit unterschiedlichem Abstand zur Austrittsöffnung vorgesehen sind.

19. Katheter nach einem der Ansprüche 1 bis 18, dadurch gekennzeichnet, dass mehrere, z.B. zwei bis fünf, vorzugsweise drei Schlingen-Drähte (5) über einen Umfangsbereich des Führungsrohres (4) zwischen 10° bis 360°, vorzugsweise 10° bis 45°, angeordnet sind.

20. Katheter nach einem der Ansprüche 1 bis 19, dadurch gekennzeichnet, dass bei mehreren Schlingen-Drähten die Befestigungsstellen (10) für diese Drähte (5, 5a) am Umfang des Führungsrohres (4) versetzt, vorzugsweise gleichmässig am Umfang verteilt sind.

21. Katheter nach einem der Ansprüch 1 bis 20, dadurch gekennzeichnet, dass ein Schlingen-Draht (5, 5b) einen von der Kreisform abweichenden Querschnitt aufweist, vorzugsweise im Querschnitt rechteckig, oval oder ellipsenförmig ausgebildet ist, und dass das Führungsrohr (4) zumindest am inneren Ende (7) bzw. an der Austrittsöffnung (8) für den Draht (5) eine entsprechende Führungsprofilierung (14) aufweist.

22. Katheter nach einem der Ansprüche 5 bis 21, dadurch gekennzeichnet, dass das innere Ende (16) des Katheters (1) gekrümmt ist und dass vorzugsweise im Übergangsbereich zu dieser Krümmung (17) eine Drainage-Öffnung (18) vorgesehen ist.

23. Katheter nach einem der Ansprüche 1 bis 22, dadurch gekennzeichnet, dass der bzw. die Schlingen-Drähte (5, 5a, 5b) beim äusseren Bedienende (11) einen oder mehrere, vorzugsweise abnehmbare, Griffe zum Manipulieren von diesen Schlingen-Drähten aufweist.

24. Katheter nach einem der Ansprüche 12 bis 23, dadurch gekennzeichnet, dass das Führungsrohr (4) zumindest im Bereich der Befestigungsöffnung (21) formstabil zum Halten des Schlingendraht-Endes ausgebildet ist.

25. Katheter nach einem der Ansprüche 1 bis 24, dadurch gekennzeichnet, dass das Führungsrohr (4) des Katheters (1) im Bereich zwischen der Befestigungsstelle (10) des Drahtes (5) und der für ihn vorgesehenen Austrittsöffnung (8) aussen am Führungsrohr eine längsverlaufende, vorzugsweise dem Querschnitt des Drahtes entsprechende, Vertiefung aufweist.

**Claims**

1. A catheter for removing calculus from the kidneys or urethra, said catheter being formed by a guide tube in whichc at least one filament is guided so as to be longitudinally slidable to form a noose for gripping a calculus from behind, the guide tube having at the inner end at least one exit for a filament, characterized in that one end of each filament (5, 5a, 5b) is fastened to the guide tube (4) in a spaced, staggered relationship to its exit (8, 8a) so that as a filament is longitudinally slid it forms a noose (9).

2. The catheter as claimed in claim 1, characterized in that the inner catheter orifice (6) forms the exit (8) for the noose filament (5, 5a, 5b).

3. The catheter as claimed in claim 1 or claim 2, characterized in that the fastening point (10) of the noose filament to the guide tube (4) is provided on the outside and is at a distance of 5 mm to 40 mm, preferably 20 mm, from the catheter orifice (6) or the exit (8, 8a).

4. The catheter as claimed in any one of claims 1 to 3, characterized in that provision is made for a plurality of noose filaments (5, 5a) preferably slidable independently of one another, and that said noose filaments may be interconnected at the fastening end on the guide tube, the point of connection being located substantially inside the catheter.

5. A catheter for removing calculus from the urethra, characterized in that the noose catheter (1) as claimed in any one of claims 1 to 4 is combined with a drainage catheter and that the filament noose (9) is located rearwardly relative to the inner drainage catheter end (16).

6. The catheter as claimed in claim 5, characterized in that a drainage conduit (19) and a guide conduit for the filament (9) are formed by a common conduit.

7. The catheter as claimed in claim 5, characterized in that the drainage conduit (19) and the guide conduit for the filament (5, 20) are separate from one another, at least in areas, in particular in the region of the exit (8a).

8. The catheter as claimed in any one of claims 1 to 7, characterized in that the noose filament(s) (5, 5a, 5b) are preferably adapted to be secured in position at the outer operating end (11).

9. The catheter as claimed in any one of claims 1 to 8, characterized in that an outer sheathing (12) of the guide tube (4) is provided for fastening the inner filament end onto the guide tube (4).

10. The catheter as claimed in any one of claims 1 to 9, characterized in that the outlet (8) provided for the noose filament (5) is located on the side of the guide tube (4) and is at a larger distance from the inner end of the catheter (1) or guide tube (4) than the fastening point (10) of the noose filament (5) on the guide tube.

11. The catheter as claimed in claim 10, characterized in that the fastening end (10) of the noose filament (5) is flexibly coupled to the guide tube (4).

12. The catheter as claimed in claim 10 or claim 11, characterized in that the fastening end of the noose filament (5) has a bend (22, 22a, 22b) for hanging into a fastening opening (21).

13. The catheter as claimed in claim 12, characterized in that the bend (22) is of hook-shaped or U-shaped configuration with a bent back hook end (24).

14. The catheter as claimed in claim 12, characterized in that the bend (22a) is of L-shaped configuration with a forwardly pointing hook end, it being preferably the case that the free, forwardly directed hook end has a length corresponding to approximately 5 to 8 times the inside diameter (d) of the guide tube (4), and that several of such bends (22a, 22a'), appropriately two, may be provided in spaced relationship to one another.

15. The catheter as claimed in claim 12, characterized in that the bend (22a) is of Z-shaped configuration with a forwardly pointing hook end (24), and that in the fastening position the hook end (24) preferably rests against the guide tube inner wall (23) opposite the fastening opening.

16. The catheter as claimed in any one of claims 1 to 15, characterized in that the fastening end of the noose filament (5) is fastened to the guide tube (4) on the outside, e.g. by means of a shrink hose possibly forming an outer sheathing of the guide tube.

17. The catheter as claimed in any one of claims 1 to 16, characterized in that the noose filament (5) is made of steel wire, possibly of plastic.

18. The catheter as claimed in any one of claims 1 to 17, characterized in that the distance between outlet (8) and fastening point (10) for the noose filament (5) or noose filaments is approximately 5 to 40 mm, preferably 20 mm, and that a plurality of fastening points may be provided at a different distance from the outlet.

19. The catheter as claimed in any one of claims 1 to 18, characterized in that a plurality of noose filaments (5), e.g. two to five, preferably three, are disposed over a peripheral area of the guide tube (4) between 10° to 360°, preferably 10° to 45°.

20. The catheter as claimed in any one of claims 1 to 19, characterized in that in cases where there are a plurality of noose filaments, the fastening points (10) for said filaments (5, 5a) are staggered over the periphery of the guide tube (4), preferably evenly distributed over the periphery.

21. The catheter as claimed in any one of claims 1 to 20, characterized in that a noose filament (5, 5b) has a cross section deviating from circularity, being preferably of rectangular, oval or ellipse-shaped configuration in cross section, and that the guide tube (4) has a corresponding guide profiling (14) at least at the inner end (7) or at the outlet (8) for the filament (5).

22. The catheter as claimed in any one of claims 5 to 21, characterized in that the inner end (16) of the catheter (1) is curved and that a drainage opening (18) is provided preferably in the transition region to said curvature (17).

23. The catheter as claimed in any one of claims 1 to 22, characterized in that the noose filament(s) (5, 5a, 5b) has (have) at the outer operating end (11) one or more grips, preferably detachable ones, for manipulating said noose filaments.

24. The catheter as claimed in any one of claims 12 to 23, characterized in that at least in the area of the fastening opening (21) the guide tube (4) is devised to be inherently stable for holding the noose filament end.

25. The catheter as claimed in any one of claims 1 to 24, characterized in that in the region between the fastening point (10) of the filament (5) and the outlet (8) provided therefor, the guide tube (4) of the catheter (1) has on the outside a recess which extends lengthwise and preferably corresponds to the cross section of the filament.

## Revendications

1. Cathéter pour l'enlèvement de calculs de la région du reinet de l'uretère, qui est formé par un tube de guidage dans lequel, pour former une boucle afin d'accrocher un calcul, au moins un fil est guidé de manière à pouvoir coulisser longitudinalement, le tube de guidage présentant du côté de l'extrémité intérieure au moins un point de sortie pour un fil, caractérisé en ce qu'une extrémité de chacun des fils (5, 5a, 5b) est fixée au tube de guidage (4) avec décalage relativement à son point de sortie (8, 8a), de sorte que lors du coulissement longitudinal d'un fil, se forme une boucle (9) formée du fil.

2. Cathéter selon la revendication caractérisé en ce que l'embouchure intérieure (6) du cathéter forme le point de sortie (8) pour le fil en boucle (5, 5a, 5b).

3. Cathéter selon l'une des revendications 1 et 2, caractérisé en ce que le point de fixation (10) du fil en boucle est prévu extérieurement sur le tube de guidage (4) et présente, relativement à l'embouchure (6) du cathéter ou au point de sortie (8, 8a), une distance de 5 à 40 mm, de préférence de 20 mm.

4. Cathéter selon l'une des revendications 1 à 3, caractérisé en ce qu'il est prévu plusieurs fils en boucle (5, 5a), pouvant, de préférence, coulisser indépendamment les uns des autres, et en ce que ces fils sont éventuellement reliés entre eux près de l'extrémité de fixation au tube de guidage, le point de liaison se trouvant essentiellement à l'intérieur du cathéter.

5. Cathéter pour l'enlèvement de calculs de l'uretère, caractérisé en ce que le cathéter à boucle (1) selon l'une des revendications 1 a 4 est combiné à un cathéter de drainage, et en ce que la boucle de fil (9) est disposée avec décalage en arrière par rapport à l'extrémité intérieure (16) du cathéter de drainage.

6. Cathéter selon la revendication 5, caractérisé en ce qu'un canal de drainage (19) et un canal de guidage du fil 9 sont formés par un canal commun.

7. Cathéter selon la revendication 5, caractérisé en ce que le canal de drainage (19) et le canal de guidage du fil (5, 20) sont séparés l'un de l'autre au moins par régions, en particulier dans la région du point de sortie.

8. Cathéter selon l'une quelconque des revendications 1 à 7, caractérisé en ce que le ou les fils en boucle (5, 5a, 5b) peuvent, de préférence, être fixés à l'extrémité extérieure de maniement (11).

9. Cathéter selon l'une quelconque des revendications 1 à 8, caractérisé en ce qu'une enveloppe extérieure (12) du tube de guidage (4) est prévue pour la fixation de l'extrémité intérieure du fil au tube de guidage (4).

10. Cathéter selon l'une quelconque des revendications 1 à 9, caractérisé en ce que l'ouverture de sortie (8) prévue pour le fil en boucle se trouve sur le côté du tube de guidage (4) et présente une plus grande distance à l'extrémité intérieure du cathéter (1) ou du tube de guidage (4) que le point de fixation (10) du fil en boucle (5) au tube de guidage.

11. Cathéter selon la revendication 10, caractérisé en ce que l'extrémité de fixation (10) du fil en boucle (5) est reliée de façon articulée au tube de guidage (4).

12. Cathéter selon l'une quelconque des revendications 10 et 11, caractérisé en ce que l'extrémité de fixation du fil en boucle (5) présente une courbure (22, 22a, 22b) pour l'accrochage dans une ouverture de fixation (21).

13. Cathéter selon la revendication 12, caractérisé en ce que la courbure (22) est constituée en forme de crochet ou en forme d'U avec extrémité de crochet recourbée (24).

14. Cathéter selon la revendication 12, caractérisé en ce que la courbure (22a) est constituée en forme de L avec extrémité de crochet tournée vers l'avant, l'extrémité libre du crochet, dirigée vers l'avant, ayant, de préférence, une longueur correspondant à 5 à 8 fois le diamètre intérieur libre (d) du tube de guidage (4), et qu'éventuellement, plusieurs, avantageusement deux ces ces courbures (22a, 22a') sont prévues avec espacement entre elles.

15. Cathéter selon la revendication 12, caractérisé en ce que la courbure est constituée en forme de Z avec extrémité de crochet (24) tournée vers l'avant, et en ce que l'extrémité de crochet (24), en position de fixation, s'applique, de préférence, contre la paroi intérieure (23) du tube de guidage qui est opposée à l'ouverture de fixation.

16. Cathéter selon l'une quelconque des revendications 1 à 15, caractérisé en ce que l'extrémité de fixation du fil en boucle (5) est fixée extérieurement au tube de guidage (4), par exemple au moyen d'une gaine rétrécissable formant éventuellement une enveloppe extérieure du tube de guidage.

17. Cathéter selon l'une quelconque des revendications 1 à 16, caractérisé en ce que le fil en boucle (5) est formé de fil d'acier, éventuellement de matière synthétique.

18. Cathéter selon l'une quelconque des revendications 1 à 17, caractérisé en ce que la distance entre l'ouverture de sortie (8) et le point de fixation (10) du fil en boucle (5) ou des fils en boucle est d'environ 5 à 40 mm, de préférence de 20 mm, et qu'éventuellement plusieurs points de fixation sont prévus avec une distance différente à l'ouverture de sortie.

19. Cathéter selon l'une quelconque des revendications 1 à 18, caractérisé en ce que plusieurs, par exemple deux à cinq, de préférence trois fils en boucle (5) sont disposés sur une région circonférentielle du tube de guidage (4) comprise entre 10° et 360°, de préférence entre 10° et 45°.

20. Cathéter selon l'une quelconque des revendications 1 à 19, caractérisé en ce que dans le cas de plusieurs fils en boucle, les points de fixation (10) de ces fils (5, 5a) à la circonférence du tube de guidage (4) sont décalés, de préférence distribués uniformément à la circonférence.

21. Cathéter selon l'une quelconque des revendications 1 à 20, caractérisé en ce qu'un fil en boucle (5, 5b) présente une section s'écartant de la forme circulaire, de préférence en ce qu'il est constitué dans sa section avec une forme rectangulaire, ovale ou elliptique, et en ce que le tube de guidage (4) présente, au moins à l'extrémité intérieure (7) ou à l'ouverture de sortie (8) du fil (5) un profil de guidage correspondant (14).

22. Cathéter selon l'une quelconque des revendications 5 à 21, caractérisé en ce que l'extrémité intérieure (16) du cathéter (1) est courbée, et en ce que, de préférence, dans la région de transition vers cette courbure (17) est prévue une ouverture de drainage (18).

23. Cathéter selon l'une quelconque des revendications 1 à 22, caractérisé en ce que le ou les fils en boucle (5, 5a, 5b) présentent, près de l'extrémité extérieure de maniement (117, une ou plusieurs poignées, de préférence amovibles, pour manipuler ces fils en boucle.

24. Cathéter selon l'une quelconque des revendications 12 à 23, caractérisé en ce que le tube de guidage (4), au moins dans la région de l'ouverture de fixation (21), est constitué avec une forme stable pour retenir l'extrémité.

25. Cathéter selon l'une quelconque des revendications 1 à 24, caractérisé en ce que le tube de guidage (4) du cathéter (1) présente, dans la région située entre le point de fixation (10) du fil (5) et l'ouverture de sortie (8) prévue pour celui-ci, extérieurement sur le tube de guidage, un creux dirigé longitudinalement, correspondant, de préférence, à la section du fil.

Fig.1 Fig.2 Fig.3 Fig.4 Fig.5 Fig.6 Fig.7 Fig.8 Fig.9

Fig.10

Fig.11

Fig.12

Fig.13

Fig.14